# EUROPÄISCHE PATENTSCHRIFT

(11) **EP 1 189 749 B1**
(45) Veröffentlichungstag und Bekanntmachung des Hinweises auf die Patenterteilung: **05.11.2003**
(21) Anmeldenummer: 00935050.5
(22) Anmeldetag: 17.05.2000
(51) Int. Cl.: B32B 7/02, A61K 9/70

(54) **VERBUNDLAMINAT UND VERFAHREN ZU SEINER HERSTELLUNG**
COMPOSITE LAMINATE AND METHOD FOR ITS PRODUCTION
STRATIFIE COMPOSITE ET SON PROCEDE DE FABRICATION

(30) Priorität: 04.06.1999 DE 19925613
(43) Veröffentlichungstag der Anmeldung: 27.03.2002
(73) Patentinhaber: LTS Lohmann Therapie-Systeme AG, 56626 Andernach (DE)
(72) Erfinder: SCHUMANN, Klaus, D-56567 Neuwied (DE)
(74) Vertreter: Schweitzer, Klaus, Dr.
(86) Internationale Anmeldenummer: EP0004459
(87) Internationale Veröffentlichungsnummer: WO00074933

(56) Entgegenhaltungen:
- EP-A- 0 307 187
- WO-A-96/19976
- DE-C- 4 237 453

## Beschreibung

Die Erfindung betrifft ein Verbundlaminat, insbesondere ein Transdermales Therapeutisches System (TTS), das in mindestens einer Schicht einen gegenüber Feuchtigkeit empfindlichen Wirkstoff enthält und das eine weitere, von dieser wirkstoffhaltigen Schicht räumlich getrennte Schicht besitzt, die zur Absorption von Feuchtigkeit befähigt ist sowie ein Verfahren zur Herstellung eines solchen Verbundlaminats.

Die transdermale Verabreichung von pharmazeutischen Wirkstoffen ist seit der ersten kommerziellen Nutzung eines scopolaminhaltigen Transdermalen Therapeutischen Systems (Scopoderm TTS) bekannt. Auch andere Wirkstoffe (Nitroglycerin, Estradiol, Clonidin, Isosorbiddinitrat, Fentanyl, Nicotin, Norethisteron etc.) werden mittlerweile in Form eines solchen TTS angeboten. Dazu werden die schichtförmig aufgebauten, wirkstoffhaltigen Pflaster auf die Haut eines Patienten aufgeklebt. Der Wirkstoff kann dann in kontrollierter Weise aus dem TTS an die Haut des Patienten abgegeben werden.

Verschiedenartige Typen solcher TTS sind z. B. von Y. W. Chien in "Developmental Concepts and Practice in Transdermal Therapeutic Systems", in *Transdermal Controlled Systemic Medications,* ed. by Y. W. Chien, Marcel Dekker, Inc., New York (1982), Kapitel 2, S. 25-81 beschrieben worden. Danach sind wesentliche Elemente eines TTS die wirkstoffundurchlässige Rückschicht, eine wirkstoffhaltige Reservoir- oder Matrixschicht, ggf. eine die Freisetzung des Wirkstoffs aus dem Reservoir kontrollierende semipermeable oder mikroporöse Membran, ggf. eine Haftkleberschicht und eine abhäsiv ausgerüstete Schutzschicht (sog. release liner), welche vor Anwendung des TTS von der haftklebend ausgerüsteten Hautkontaktseite des TTS entfernt wird. Um Wiederholungen zu vermeiden, soll der Inhalt - insbesondere die darin beschriebenen TTS-Typen - dieses Kapitels durch Rückbeziehung als Teil der Offenbarung der vorliegenden Beschreibung gelten. Ein TTS ist somit aufgrund seines schichtförmigen Aufbaus ein Verbundlaminat, also eine aus mindestens zwei Schichten aufgebaute Vorrichtung.

Ein Problem, das bei der Herstellung und Lagerung von TTS auftreten kann, beruht auf der Empfindlichkeit einzelner Komponenten, insbesonders bestimmter Wirkstoffe gegenüber Feuchtigkeit. Hierunter ist zu verstehen, daß bei Einwirkung schon von geringen Mengen von Wasser eine physikalische und / oder chemische Umwandlung der Komponente und / oder des Wirkstoffs erfolgen kann, die dazu führt, dass diese Komponente ihre strukturelle Unversehrtheit verlieren kann bzw. dass dieser Wirkstoff nicht mehr in seiner vollen Aktivität für die pharmazeutische Anwendung zur Verfügung steht.

Beispiele für Stoffe, insbesondere pharmazeutische Wirkstoffe, die bei Einwirkung von Feuchtigkeit die oben genannten Umwandlungen erleiden können, sind Acetylsalicylsäure (ASS) und 17β-Estradiol. Im Fall von Acetylsalicylsäure erfolgt eine chemische Umsetzung (Hydrolyse), wobei Salicylsäure und Essigsäure gebildet werden. Im Fall von 17β-Estradiol erfolgt eine physikalische Umwandlung (Kristallbildung), wobei schwerlösliche wasserhaltige Kristalle gebildet werden. Als Folge dieser Umwandlungen liegt der pharmazeutische Wirkstoff in den verschiedenen Schichten des TTS, insbesondere der Matrixschicht, des Reservoirs und / oder der Haftkleberschicht in einer geringeren Konzentration gelöst vor. Im allgemeinen ist jedoch eine hohe Konzentration von gelöstem Wirkstoff in den verschiedenen Schichten des TTS - insbesondere der Hautkontaktschicht - gewünscht, um über die gesamte Anwendungsdauer des TTS eine ausreichend große Konzentrationsdifferenz zwischen Hautkontaktschicht und der Haut des Patienten zu gewährleisten. Diese Konzentrationsdifferenz ist nämlich die treibende Kraft für die Diffusion des Wirkstoffs in die Haut und den Blutkreislauf.

Um das Problem der Kristallbildung des Wirkstoffs infolge der Feuchtigkeitseinwirkung zu vermeiden, sind verschiedene Methoden und Vorrichtungen entwickelt worden, um den dafür verantwortlichen Feuchtigkeitsgehalt in der das TTS enthaltenden Vorrichtung - hierbei handelt es sich um die Primärverpackung, üblicherweise um einen versiegelten Beutel - zu reduzieren.

So wurde in DE 42 37 453 vorgeschlagen, die Transdermalen Therapeutischen Systeme, die als feuchtigkeitsempfindlichen Wirkstoff 17β-Estradiol enthalten, unter Bedingungen mit reduzierter Luftfeuchtigkeit herzustellen und zu lagern. Dazu wurde ein Trockenmittel in Form von mehreren Körnchen Blaugel zusätzlich zu dem TTS in den versiegelten Beutel gegeben.

In der DE 196 46 048 sind nun Verpackungen für Transdermale Therapeutische Systeme beschrieben, die eine innenseitig fixierte Trocknungsvorrichtung enthält. Die Trocknungsvorrichtung ist flächenförmig ausgestaltet und enthält ein in fester Form vorliegendes Trockenmittel. Hergestellt werden diese Trocknungsvorrichtungen in einem mehrstufigen Verfahren. Dazu muß in einem ersten Arbeitsschritt das Trockenmitteletikett durch Vereinzelung aus einem bahnförmigen Trockenmittellaminat hergestellt, in einem zweiten Arbeitsschritt mit einer Zwischenabdeckung versehen und in einem dritten Arbeitsschritt unter Entfernung dieser abhäsiv ausgerüsteten Zwischenabdeckung exakt auf die Bahn, die die spätere Innenseite des Siegelbeutels ergibt, positioniert und dauerhaft festgeklebt werden. In einem weiteren, separaten Arbeitsgang muß das TTS mit dem betreffenden Wirkstoff hergestellt werden. Schließlich muß dieses TTS auf die mit den einzelnen Trockenmitteletiketten beklebte Siegelbeutellaminatbahn aufgelegt werden und diese Bahn so geschnitten und versiegelt werden, daß das Trockenmitteletikett nicht auf den dabei entstehenden Schweißnähten liegt. Die Durchführung dieser verschiedenen Arbeitsschritte und Verfahren muß unter Ausschluß von bzw. bei stark reduzierter Luftfeuchtigkeit erfolgen, was gegebenenfalls zusätzlich ein zügiges Arbeiten erfordert. Dies ist jedoch wegen der erforderlichen Genauigkeit bei der Positionierung des Trockenmitteletiketts auf dem Laminat bzw. der Positionierung des TTS auf dem so beklebten Laminat technisch sehr anspruchsvoll und / oder apparativ sehr aufwendig.

Aufgabe der vorliegenden Erfindung ist es daher, eine Vorrichtung zur Verfügung zu stellen, durch die ein Verbundlaminat, insbesondere ein Transdermales Therapeutisches System (TTS), welches in mindestens einer Schicht eine gegenüber Feuchtigkeit empfindliche Komponente, insbesondere einen feuchtigkeitsempfindlichen Wirkstoff enthält, in einem abgeschlossenen Gasraum unter reduzierter Luftfeuchtigkeit gelagert werden kann, sowie ein Verfahren zur Herstellung einer solchen Vorrichtung. Dabei sollen Vorrichtungen mit einfacherem Aufbau und Verfahren, die die dargestellten Schwierigkeiten bei der Herstellung der im Stand der Technik bekannten Vorrichtungen vermeiden, zur Verfügung gestellt werden.

Gelöst wird diese Aufgabe gemäß der vorliegenden Erfindung durch ein Verbundlaminat wie definiert in Anspruch 1. In dem erfindungsgemäßen Verbundlaminat ist mindestens eine feuchtigkeitsempfindliche Schicht (1) enthalten, welche eine gegenüber Feuchtigkeit empfindliche Komponente enthält. In dem erfindungsgemäßen Verbundlaminat ist weiterhin eine wirkstoffreie, feuchtigkeitsabsorbierende Schicht (2) enthalten, die zur Absorption von Feuchtigkeit befähigt ist. Die beiden separaten Schichten sind räumlich voneinander getrennt. Bei dem Verbundlaminat kann es sich dabei bevorzugt um ein Transdermales Therapeutisches System mit mindestens einem feuchtigkeitsempfindlichen Wirkstoff handeln.

Die erfindungsgemäße Vorrichtung ist dadurch gekennzeichnet, daß sie mindestens eine feuchtigkeitsempfindliche Schicht (1) enthält. Die Feuchtigkeitsempfindlichkeit dieser Schicht beruht darauf, daß eine feuchtigkeitsempfindliche Komponente darin enthalten ist. Dazu ist einerseits ein feuchtigkeitsempfindliches Material zu zählen, aus dem die Schicht aufgebaut ist und die somit im wesentlichen eine Trägerfunktion besitzen. Solche Materialien bestehen im allgemeinen aus einem oder mehreren polymeren Basismaterialien.

Hierunter ist ein Material zu verstehen, das aus solchen Molekülen aufgebaut ist, in denen eine Art oder mehrere Arten von Atomen oder Atomgruppierungen wiederholt aneinandergereiht sind. Zu feuchtigkeitsempfindlichen polymeren Basismaterialien können z. B. Polyvinylacetat, Polyvinylalkohol, Polyvinylpyrrolidon, Polyanhydride, Biopolymere, Cellulose, Celluloseprodukte, sog. in Wasser quellbare Polymere und sonstige makromolekulare Stoffe zählen, die sich bei Einwirkung von Feuchtigkeit (im wesentlichen Wasser) lösen, quellen oder chemisch umwandeln.

Im allgemeinen ist die Komponente, die die Feuchtigkeitsempfindlichkeit dieser Schicht verursacht, jedoch mindestens ein in dieser Schicht enthaltener feuchtigkeitsempfindlicher Stoff. Dieser Stoff besitzt in dem erfindungsgemäßen Verbundlaminat keine besondere Trägerfunktion, sondern stellt das eigentlich zu tragende wirksame Prinzip dar. Zu solchen feuchtigkeitsempfindlichen Stoffen können auch pharmazeutische Wirkstoffe zählen, insbesondere die schon genannten Wirkstoffe Acetylsalicylsäure und 17β-Estradiol, aber auch Nicotin, Morphin, Diamorphin, Lobelin, Norethisteronacetat, Fentanyl, Lobelin, Selegilin, Testosteron und hydrolyseempfindliche Ester. Zu den feuchtigkeitsempfindlichen Stoffen können aber auch Stoffe mit einem relativ hohen Molekulargewicht zählen, wie z. B. Insulin und weitere bekannte Eiweißstoffe (Proteine).

Die erfindungsgemäße Vorrichtung enthält weiterhin eine wirkstofffreie, feuchtigkeitsabsorbierende Schicht (2). Eine solche zur Absorption von Feuchtigkeit befähigte Schicht kann z. B. aus mindestens einem polymeren Material bestehen, das feuchtigkeitsabsorbierend ist oder ein Trockenmittel enthält. Zu den geeigneten polymeren Materialien sind solche zu zählen, die feuchtigkeitsdurchlässig sind. Feuchtigkeitsabsorbierende polymere Materialien sind z. B.: Polymere mit einer darin enthaltenen N=C=N -Einheit (das Carbodiimid-Strukturelement).

Man kann aber auch polymere Materialien verwenden, die nicht zur Feuchtigkeitsabsorption befähigt sind, sondern nur als Trägermaterial für das eigentliche Trockenmittel dienen. Zu solchen polymeren Materialien gehören Polyethylen, Polypropylen, Polyacrylat, Polyisobuten, Polystyrol, Polyvinylchlorid, Polyvinylidenchlorid, Synthesekautschuk, Silikone, Ethylen-Vinylacetat, und andere, dem Fachmann bekannte Thermoplasten und Elastomere.

Zu den geeigneten Trockenmitteln zählen insbesondere Feststoffe. Hierzu gehören mineralische Stoffe wie Silicagel, Molekularsieb, Calciumsulfat (CaSO₄, Anhydrit), Aluminiumoxid Al₂O₃, Calciumoxid CaO, Kaliumcarbonat K₂CO₃, Natriumcarbonat Na₂CO₃, Natriumsulfat Na₂SO₄, und weitere, als Trockenmittel geeignete und dem Fachmann bekannte Feststoffe sowie Mischungen hiervon.

Das feuchtigkeitsabsorbierende polymere Material oder das Trockenmittel befindet sich in der feuchtigkeitsabsorbierenden Schicht (2) in einem aktiven Zustand, in dem das darin enthaltene feuchtigkeitsabsorbierende polymere Material oder das Trockenmittel zur Aufnahme von Feuchtigkeit (Wassermolekülen) befähigt ist. Das Trockenmittel muß gegebenenfalls zuvor aus einem inaktiven Zustand, in dem es nicht zur Aufnahme von Feuchtigkeit befähigt ist, in diesen aktiven Zustand überführt werden. Das feuchtigkeitsabsorbierende polymere Material und / oder das Trockenmittel selbst ist nicht oder nur in sehr geringem Ausmaß zum Heraustreten (Diffusion) aus der feuchtigkeitsabsorbierenden Schicht befähigt, d. h. es ist in dieser Schicht praktisch immobilisiert.

Die feuchtigkeitsabsorbierende Schicht (2) kann in bestimmten Ausführungsformen zweckmäßigerweise haftklebend sein, sie kann aber in bestimmten Ausführungen auch nicht haftklebend sein oder zumindest nur einseitig haftklebend. Um die feuchtigkeitsabsorbierende Schicht (2) haftklebend auszurüsten, müssen als polymeres Material gegebenenfalls haftklebende Polymere oder nichthaftklebende Polymere unter Zusatz von Klebrigmachern (engl.: tackifier) verwendet werden. Haftklebende Polymere und / oder nichthaftklebende Polymere mit geeigneten Klebrigmachern sind dem Fachmann bekannt. Zu haftklebenden Polymeren zählen z. B. Polyacrylate, Polyisobuten, Silikone; Klebrigmacher sind z. B. Harze wie Kolophonium.

Um die feuchtigkeitsabsorbierende Schicht (2) nur einseitig haftklebend auszurüsten, muß eine nichthaftende Schicht auf eine Seite der haftklebend ausgerüsteten feuchtigkeitsabsorbierenden Schicht auflaminiert werden.

Der Feuchtigkeitsgehalt der feuchtigkeitsempfindlichen Schicht (1) kann durch das gleichzeitige Vorliegen der feuchtigkeitsabsorbierenden Schicht (2) in der Vorrichtung konstant und / oder unter eines bestimmten gewünschten Grenzwertes gehalten werden. In einer bevorzugten Ausführungsform liegt der Feuchtigkeitsgehalt der feuchtigkeitsempfindlichen Schicht (2) unterhalb von 0,5 Gew.-%, in einer besonders bevorzugten Ausführungsform liegt der Feuchtigkeitsgehalt der feuchtigkeitsempfindlichen Schicht (2) des Verbundlaminats unterhalb von 0,2 Gew.-%.

Die räumliche Trennung dieser beiden unterschiedlichen Schichten (1) und (2) innerhalb des Verbundlaminats ist ein weiteres Kennzeichen des Verbundlaminats. Diese räumliche Trennung der beiden unterschiedlichen Schichten kann auf verschiedene Weise realisiert werden. In einem Fall existiert eine horizontale Trennschicht (3) zwischen den beiden Schichten, im anderen Fall eine vertikale Trennschicht (4).

Diese Trennschicht besitzt die Funktion, das Eintreten von Trockenmittel in die feuchtigkeitsempfindliche Schicht (1) und / oder das Eintreten der feuchtigkeitsempfindlichen Komponente in die feuchtigkeitsabsorbierende Schicht (2) zu verhindern. In dem Fall, daß aber das Eintreten von feuchtigkeitsabsorbierendem polymeren Material und / oder vom Trockenmittel in die feuchtigkeitsempfindliche Schicht (1) und / oder das Eintreten der feuchtigkeitsempfindlichen Komponente in die feuchtigkeitsabsorbierende Schicht (2) praktisch nicht stattfindet und / oder zumindest keine nachteiligen Effekte auf die jeweils betroffene Schicht bewirkt, kann auf die Verwendung einer solchen zusätzlichen Trennschicht verzichtet werden. In einer solchen Ausführungsform können die feuchtigkeitsempfindliche Schicht (1) und die feuchtigkeitsabsorbierende Schicht (2) in zwei separaten Schichten benachbart nebeneinander, insbesondere aufeinanderliegend vorliegen und direkten Kontakt zueinander besitzen.

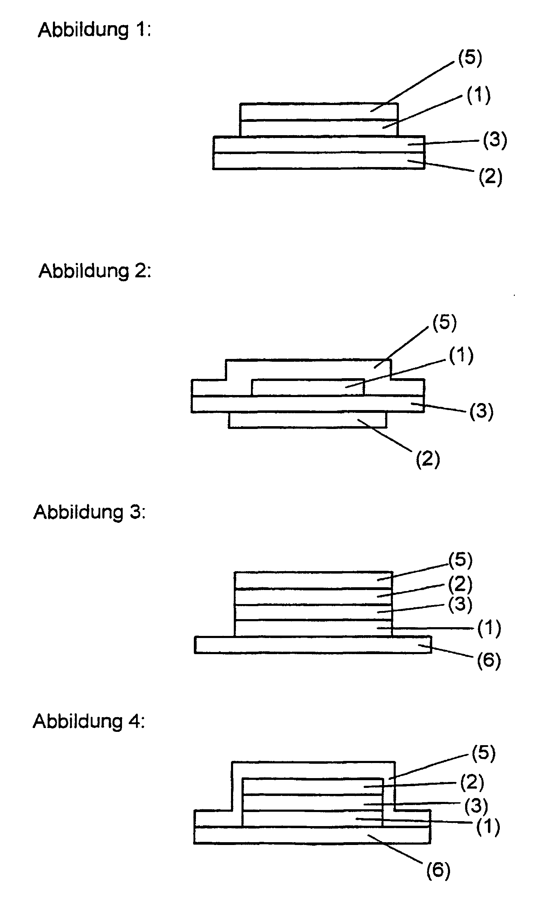

Eine konkrete Ausführungsformen für eine horizontale Trennschicht (3) ist eine Schutzschicht, die unterhalb der feuchtigkeitsempfindlichen Schicht (1) und oberhalb der feuchtigkeitsabsorbierenden Schicht (2) angeordnet ist. Dieser Fall stellt eine sehr einfache und daher bevorzugte Ausführungsform dar. Er ist in Abbildung 1 wiedergegeben. Die Abbildung 1 zeigt ein Verbundlaminat mit horizontaler Trennschicht (3), einer einseitig haftklebenden feuchtigkeitsabsorbierenden Schicht (2) und einer seitlich offenen feuchtigkeitsempfindlichen Schicht (1). Die horizontale Trennschicht (3) dient zugleich als gemeinsame Trägerschicht von feuchtigkeitsempfindlicher Schicht (1) und feuchtigkeitsabsorbierender Schicht (2) sowie als Schutzschicht (release liner) für die feuchtigkeitsempfindliche Schicht (1). Sie ist somit mit der Schutzschicht eines konventionellen TTS identisch. Die feuchtigkeitsempfindliche Schicht (1) wird nach oben von der Rückschicht (5) abgedeckt, die wiederum feuchtigkeitsdurchlässig oder feuchtigkeitsundurchlässig sein kann.
Die feuchtigkeitsabsorbierende Schicht (2) ist bevorzugt kongruent (flächengleich) zu der horizontalen Trennschicht (3), sie kann aber auch kleiner als die Fläche der horizontalen Trennschicht (3) sein. In dieser Ausführungsform ist die feuchtigkeitsabsorbierende Schicht (2) einseitig haftklebend ausgerüstet, so daß sie mit der horizontalen Trennschicht (3) fest verbunden ist und erst unmittelbar vor Applikation der restlichen Schichten des Verbundlaminats (d. h. des eigentlich zu applizierenden TTS) gemeinsam mit dieser, gleichzeitig als Schutzschicht dienenden Schicht abgezogen wird. Die horizontale Trennschicht (3) kann undurchlässig für Feuchtigkeit sein; bevorzugt ist jedoch eine für Feuchtigkeit durchlässige, zugleich für die feuchtigkeitsempfindliche Komponente jedoch undurchlässige Ausführungsform. Bei Verwendung einer feuchtigkeitsundurchlässigen horizontalen Trennschicht (3) verwendet man vorzugsweise eine feuchtigkeitsdurchlässige Rückschicht (5).

Für den Fall, daß die Rückschicht (5) feuchtigkeitsundurchlässig ist und daß sie gleichzeitig die feuchtigkeitsempfindliche Schicht (1) vollständig, also auch seitlich gegenüber der Umgebung abschließt, verwendet man eine solche feuchtigkeitsdurchlässige horizontale Trennschicht (3), die gleichzeitig die Schutzschicht (6) darstellt. Ein solches Verbundlaminat ist in Abbildung 2 dargestellt.

In einer weiteren Ausführungsform besteht die horizontale Trennschicht (3) aus einer Zwischenschicht, die oberhalb der feuchtigkeitsempfindlichen Schicht (1) und unterhalb der feuchtigkeitsabsorbierenden Schicht (2) angeordnet ist. Im Unterschied zu den in den Abbildungen 1 und 2 dargestellten Ausführungsformen muß bei dieser Ausführungsform eine Abtrennung der feuchtigkeitsabsorbierenden Schicht (2) unmittelbar vor der Applikation des TTS nicht zwangsläufig erfolgen. Dies hängt vom Aufbau des Verbundlaminats ab.

Wenn die horizontale Trennschicht (3) auf der zur feuchtigkeitsabsorbierenden Schicht (2) gewandten Seite abhäsiv ausgerüstet ist oder wenn die zur horizontalen Trennschicht (3) gewandte Seite der feuchtigkeitsabsorbierenden Schicht (2) abhäsiv ausgerüstet ist, kann eine Abtrennung der feuchtigkeitsabsorbierenden Schicht (2) einschließlich der Rückschicht (5) erfolgen. Dieser Fall ist schematisch in Abbildung 3 dargestellt. In diesem Fall kann die feuchtigkeitsabsorbierende Schicht (2) gegebenenfalls auch mit der Rückschicht (5) des Verbundlaminats identisch sein.

Wenn jedoch weder die horizontale Trennschicht (3) noch die feuchtigkeitsabsorbierende Schicht (2) zumindest einseitig abhäsiv ausgerüstet sind und / oder wenn der Verbund dieser beiden Schichten durch eine gemeinsame Rückschicht (5) abgedeckt ist, die den Verbund dieser Schichten zur Umgebung hin vollständig abschließt, kann keine Abtrennung der feuchtigkeitsabsorbierenden Schicht (2) erfolgen. Diesen Fall zeigt Abbildung 4. Das dargestellte Verbundlaminat besitzt eine horizontale Trennschicht (3), die eine feuchtigkeitsdurchlässige Zwischenschicht innerhalb der Vorrichtung darstellt. Rückschicht (5) und Schutzschicht (6) schließen den Verbund aus feuchtigkeitsabsorbierender Schicht (2), horizontaler Trennschicht (3) und feuchtigkeitsempfindlicher Schicht (1) gegenüber der Umgebung vollständig ab. In einer solchen Ausführungsform, bei der die feuchtigkeitsabsorbierende Schicht (2) zusammen mit den restlichen Schichten der Vorrichtung appliziert wird, kann es vorkommen, daß das darin enthaltene Trockenmittel während der Dauer der Anwendung durch Wasseraufnahme in den inaktiven Zustand zurückversetzt wird.

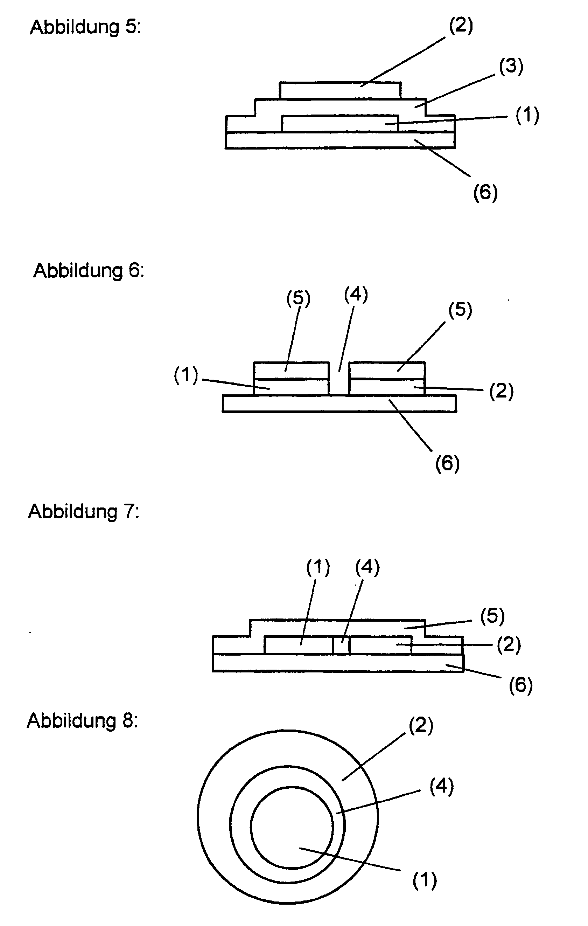

In Abbildung 5 ist gezeigt, daß die feuchtigkeitsabsorbierende Schicht (2) auch oberhalb der konventionellen Rückschicht angebracht sein kann. In diesem Fall ist die horizontale Trennschicht (3) mit einer solchen Rückschicht identisch. Sofern die zur feuchtigkeitsabsorbierenden Schicht (2) gewandte Seite der horizontalen Trennschicht (3) oder die zur horizontalen Trennschicht (3) gewandte Seite der feuchtigkeitsabsorbierenden Schicht (2) abhäsiv ausgerüstet ist, kann eine Abtrennung dieser Schicht (2) von der Trennschicht (3) vor der Applikation des TTS erfolgen.

Der Fall einer vertikalen Trennschicht (4) wird bevorzugt dann eingesetzt, wenn ein besonders flache Vorrichtung hergestellt werden soll oder keine geeigneten Materialien (z. B. ein feuchtigkeitsdurchlässiges, aber gegenüber den feuchtigkeitsempfindlichen Komponenten der feuchtigkeitsempfindlichen Schicht undurchlässiges Material) für eine horizontale Trennschicht zur Verfügung stehen.

Die einfachste Ausführungsform des erfindungsgemäßen Verbundlaminats, die eine vertikale Trennschicht (4) enthält, ist in Abbildung 6 dargestellt. In dieser Ausführungsform besteht die vertikale Trennschicht (4) aus einer Materialaussparung (Lücke; luftgefüllter, ansonsten leerer Raum) zwischen der feuchtigkeitsempfindlichen Schicht (1) und der feuchtigkeitsabsorbierenden Schicht (2). Die Rückschichten (5) können gleich oder verschieden sein.

Die vertikale Trennschicht (4) kann aber auch aus einem festen Material bestehen, das für Feuchtigkeit durchlässig und gleichzeitig gegenüber den feuchtigkeitsempfindlichen Komponenten der feuchtigkeitsempfindlichen Schicht (1) im wesentlichen undurchlässig ist. Abbildung 7 zeigt ein solches Verbundlaminat mit vertikaler Trennschicht (4) aus einem solchen Material. Die Rückschicht (5) schließt die feuchtigkeitsempfindliche Schicht (1) und die feuchtigkeitsabsorbierende Schicht (2) gegenüber der Umgebung vollständig ab.

In den Ausführungsformen mit einer vertikalen Trennschicht (4) haften feuchtigkeitsempfindliche Schicht (1) und feuchtigkeitsabsorbierende Schicht (2) auf derselben Seite einer einzigen Schutzschicht (6).

Die feuchtigkeitsempfindliche Schicht (1), die feuchtigkeitsabsorbierende Schicht (2) und die vertikale Trennschicht (4) können verschiedene Rückschichten (5) oder die gleiche Rückschicht (5) besitzen.

In den Ausführungsformen mit vertikaler Trennschicht (4) ist die geometrische Form von feuchtigkeitsabsorbierender Schicht (2) und / oder feuchtigkeitsempfindlicher Schicht (1) grundsätzlich egal. Bevorzugt ist eine rechteckige oder quadratische Form.

Es ist möglich, daß die feuchtigkeitsabsorbierende Schicht (2) vor der Applikation des eigentlichen TTS gemeinsam mit der Schutzschicht (6) von den übrigen Schichten des Verbundlaminats abgetrennt wird. Dies ist z. B. in der Ausführungsform von Abbildung 6 möglich. Wenn eine Ausführungsform wie in Abbildung 7 verwendet wird, ist dies nicht möglich.

In Abbildung 8 ist eine weiteres Verbundlaminat wiedergegeben, bei der die feuchtigkeitsabsorbierende Schicht (2) ringförmig um eine zentrale kreisförmige feuchtigkeitsempfindliche Schicht (1) angeordnet ist, die durch eine ringförmige vertikale Trennschicht (4) getrennt ist. Es ist klar, daß in einer solchen Weise grundsätzlich auch die feuchtigkeitsempfindliche Schicht (1) ringförmig um eine kreisförmige feuchtigkeitsabsorbierende Schicht (2) angeordnet sein kann.

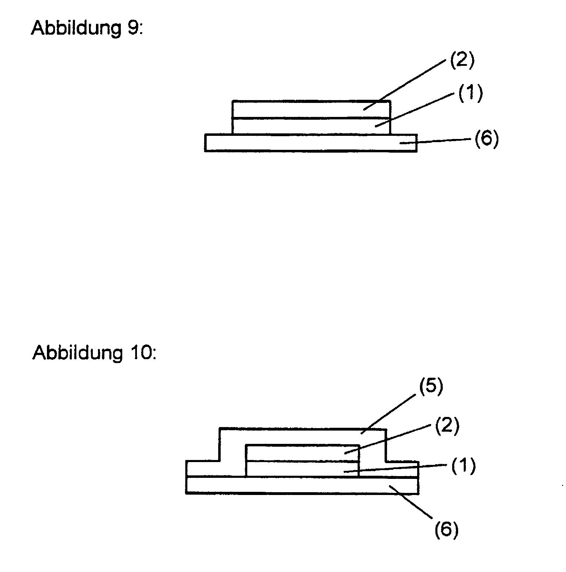

Abbildung 9 zeigt ein Verbundlaminat, bei der die feuchtigkeitsempfindliche Schicht (1) und die feuchtigkeitsabsorbierende Schicht (2) in zwei separaten Schichten benachbart nebeneinander vorliegen und direkten Kontakt zueinander besitzen. Auf eine zusätzliche Trennschicht dazwischen wurde aufgrund der Abwesenheit der zuvor beschriebenen Wechselwirkungen, die im wesentlichen durch Eindiffundieren von Inhaltsstoffen der einen Schicht in die jeweils andere Schicht verursacht werden, verzichtet.

Abbildung 10 zeigt eine Ausführungsform wie in Abbildung 9, jedoch ist der Verbund aus feuchtigkeitsempfindlicher Schicht (1) und feuchtigkeitsabsorbierender Schicht (2) zusätzlich von einer Rückschicht (5) abgedeckt. Hierdurch wird ein vollständiger Abschluß von feuchtigkeitsempfindlicher Schicht (1) und feuchtigkeitsabsorbierender Schicht (2) gegenüber der Umgebung erzielt.

Die üblichen weiteren Komponenten eines TTS (Rückschicht, mikorporöse und /oder semipermeable Membran, zusätzliche Haftkleberschicht, Vlies, etc.) können gegebenenfalls in allen besprochenen und dargestellten Ausführungsformen um die feuchtigkeitsempfindliche Schicht (1) herum angeordnet oder in ihr selbst enthalten sein.

Das erfindungsgemäße Verfahren, definiert in Anspruch 7, besteht darin, daß die mindestens eine feuchtigkeitsempfindliche Schicht (1) und die mindestens eine feuchtigkeitsabsorbierende Schicht (2) auf eine Trägerschicht aufgebracht werden. Das Verfahren erfolgt dabei in mehreren Schritten, wobei die zwei zentralen Verfahrensschritte in dem Zusammenbringen der feuchtigkeitsempfindlichen Schicht (1) mit einer Trägerschicht und in dem Zusammenbringen der feuchtigkeitsabsorbierenden Schicht (2) mit einer Trägerschicht zu sehen ist.

Diese zwei zentralen Verfahrensschritte können erfindungsgemäß zeitlich und /oder räumlich aufeinanderfolgend stattfinden. Die zwei zentralen Verfahrensschritte können aber auch zeitgleich stattfinden und / oder in räumlicher Nähe. Unter räumlicher Nähe ist zu verstehen, daß die beiden Schichten unmittelbar nebeneinanderliegend (d. h. im Abstand der vertikalen Trennschicht) auf dieselbe Trägerschicht aufgelegt werden. Unter räumlicher Nähe ist auch zu verstehen, daß die beiden Schichten direkt gegenüberliegend auf die Ober- und Unterseite derselben Trägerschicht aufgelegt werden.

Als Trägerschicht kann in den Ausführungsformen mit einer horizontalen Trennschicht die horizonale Trennschicht selbst sein und in den Ausführungsformen mit einer vertikalen Trennschicht eine gemeinsame Trägerschicht, z. B. ein als Schutzschicht dienender release liner.

Die Herstellung der feuchtigkeitsempfindlichen Schicht (1) ist dem Fachmann grundsätzlich bekannt und kann auf konventionelle Weise erfolgen, z. B.: Mischen der Komponenten wie polymeres Material, Wirkstoff, ggf. Klebrigmacher, Weichmacher, Lösungsmittel, Vernetzer etc. und Ausstreichen der Mischung und Abziehen des Lösungsmittels oder Extrusion bei lösungsmittelfreien Mischungen etc. In den Komponenten dieser Mischung ist natürlich auch die feuchtigkeitsempfindliche Komponente enthalten.

Auch die Herstellung einer feuchtigkeitsabsorbierenden Schicht (2) ist grundsätzlich bekannt. Sie kann auf denselben konventionellen Weisen erfolgen wie die Herstellung der feuchtigkeitsempfindlichen Schicht (1). Es werden jedoch mindestens ein feuchtigkeitsabsorbierendes polymeres Material und / oder einTrockenmittel mit gegebenenfalls mindestens einem Matrixmaterial gemischt. Um eine haftklebende feuchtigkeitsabsorbierende Schicht zu erhalten, muß gegebenenfalls das Matrixmaterial haftklebend sein oder ein Klebrigmacher (tackifier) der Mischung zugesetzt werden. Diese Mischung wird dann auf einer Zwischenabdeckung ausgestrichen. Um diese haftklebende feuchtigkeitsabsorbierende Schicht gegebenenfalls nur einseitig haftklebend auszurüsten, verwendet man zusätzlich eine nichtklebende Endabdeckung.

Ein weiterer Verfahrensschritt kann das Aktivieren der feuchtigkeitsabsorbierenden Schicht (2) sein. Hierunter ist zu verstehen, daß das in dieser Schicht enthaltene Trockenmittel aus einem inaktiven Zustand, der nicht zur Aufnahme von Feuchtigkeit befähigt, in einen aktiven Zustand, der zur Aufnahme von Feuchtigkeit befähigt, überführt wird. Dies kann auf verschiedene Weise geschehen, z. B. durch Erwärmen auf erhöhte Temperaturen und / oder Lagern unter extrem trockenen Bedingungen und / oder stark reduziertem Außendruck. Weitere Methoden und Bedingungen zur Aktivierung des Trockenmittels sind dem Fachmann bekannt. Die Aktivierung der feuchtigkeitsabsorbierenden Schicht (2) kann erfolgen, bevor das Laminats aus feuchtigkeitsabsorbierender Schicht (2), Trägerschicht und feuchtigkeitsempfindlicher Schicht (1) hergestellt ist. Sie kann aber auch nach der Herstellung dieses Laminats erfolgen, sofern die Bedingungen der Aktivierung keine nachteiligen Auswirkungen auf die feuchtigkeitsempfindliche Schicht (1) oder andere Bestandteile dieses Verbundlaminats ausüben. Es ist klar, daß nach der Aktivierung des Trockenmittels bzw. bei Verwendung eines feuchtigkeitsabsorbierenden polymeren Materials die weiteren sich anschließenden bzw. alle Verfahrensschritte möglichst unter Ausschluß bzw. bei stark reduzierter Luftfeuchtigkeit und / oder besonders zügig durchgeführt werden sollten.

Weitere Verfahrensschritte bei der Herstellung eines erfindungsgemäßen Verbundlaminats können die folgenden sein: Aufkaschieren weiterer Bestandteile wie z. B.: einer Rückschicht (5), einer zusätzlichen Vliesstoffschicht, einer semipermeablen oder einer mikroporösen Membran, einer zusätzlichen Haftkleberschicht, weitere Schutzschichten auf die feuchtigkeitsempfindliche Schicht (1) und / oder die feuchtigkeitsabsorbierende Schicht (2).

Der Zeitpunkt, wann diese weiteren Verfahrensschritte ausgeführt werden, hängt hinsichtlich der zentralen Verfahrensschritte davon ab, welchen Aufbau das erfindungsgemäße Verbundlaminat letztendlich erhalten soll. Dem Fachmann ist bekannt, ob ein entsprechender Verfahrensschritt vor, gleichzeitig oder nach den zentralen Arbeitsschritten durchzuführen ist. So kann z. B. das Anbringen einer gemeinsamen zusätzlichen Rückschicht (5) nach Herstellung des Verbundlaminats aus feuchtigkeitsempfindlicher Schicht (1) und feuchtigkeitsabsorbierender Schicht (2) vorteilhaft sein, während das Anbringen einer Membran auf die feuchtigkeitsempfindliche Schicht (1) vor Herstellung des Verbunds enthaltend feuchtigkeitsempfindliche Schicht (1) und feuchtigkeitsabsorbierende Schicht (2) zweckmäßiger sein kann.

Abschließende Verfahrensschritte sind das Vereinzeln des Verbundlaminats durch Schneiden oder Stanzen und das Einbringen der so vereinzelten Muster (Stanzlinge) des Verbundlaminats (insbesondere von Einzel-TTS) in entsprechende Siegelbeutel oder Tiefziehblister sein.

Die zugrundeliegende Aufgabe wird durch die vorliegende Erfindung in überraschender und besonders einfacher Weise gelöst. Ein Vorteil besteht darin, daß die feuchtigkeitsabsorbierende Schicht nun direkt in der Vorrichtung enthalten ist, die auch die feuchtigkeitsempfindliche Schicht enthält. Durch die dargestellten Ausführungsformen werden auch mögliche Inkompatibilitätsprobleme zwischen den Inhaltsstoffen der feuchtigkeitsempfindlichen Schicht und denen der feuchtigkeitsabsorbierenden Schicht weitgehend vermieden. Das erfindungsgemäße Verfahren ist aufgrund des Wegfalls von separaten Herstellungsvorgängen für das trockenmittelhaltige Laminat und das wirkstoffhaltige Laminat vereinfacht.

Das folgende Beispiel soll die Herstellung eines Verbundlaminats näher erläutern. Beispiel 1:

Auf einer ersten Vorratsrolle (7) befindet sich eine konventionell hergestellte, CaSO₄-haltige, einseitig haftklebende Schicht (9), welche mit einer abhäsiv ausgerüsteten Schutzfolie (10) abgedeckt ist. Auf einer zweiten Vorratsrolle (8) befindet sich eine den Wirkstoff 17β-Estradiol als feuchtigkeitsempfindlicher Komponente enthaltende Schicht (11). Diese feuchtigkeitsempfindliche Schicht (11) ist ebenfalls haftklebend und mit einer Rückschicht (12) sowie einer abhäsiv ausgerüsteten Zwischenabdeckung (13) ausgerüstet.

Das auf der ersten Vorratsrolle (7) befindliche Laminat wird abgerollt. Von dem auf der zweiten Vorratsrolle (8) befindlichen Laminat wird die Zwischenabdeckung (13) abgezogen und die haftklebend ausgerüstete Seite der feuchtigkeitsempfindlichen Schicht (11) auf die Rückseite der Schutzfolie (10) des von der ersten Vorratsrolle (8) abgerollten Laminats aufgebracht

An einer folgenden Hubstanze (14) werden die Konturen des Pflasters aus dem Laminat aus Rückschicht (12) und feuchtigkeitsempfindlicher Schicht (11) ausgestanzt. Der überstehende Rand wird abgegittert. An einer weiteren Stanzvorrichtung werden auf die Schutzfolie (10) Noppen und / oder Abziehhilfen in Form von Schnitten eingestanzt.

Schließlich wird die Schutzfolie, gegebenenfalls zusammen mit der trockenmittelhaltigen Schicht an einer weiteren Stanzvorrichtung (15), z. B. einer Hubstanze, durchtrennt Die durch ein solches Querschneiden vereinzelten Verbundlaminate werden auf eine Bahn aus einem siegelfähigen Material gelegt und in konventioneller Weise versiegelt.

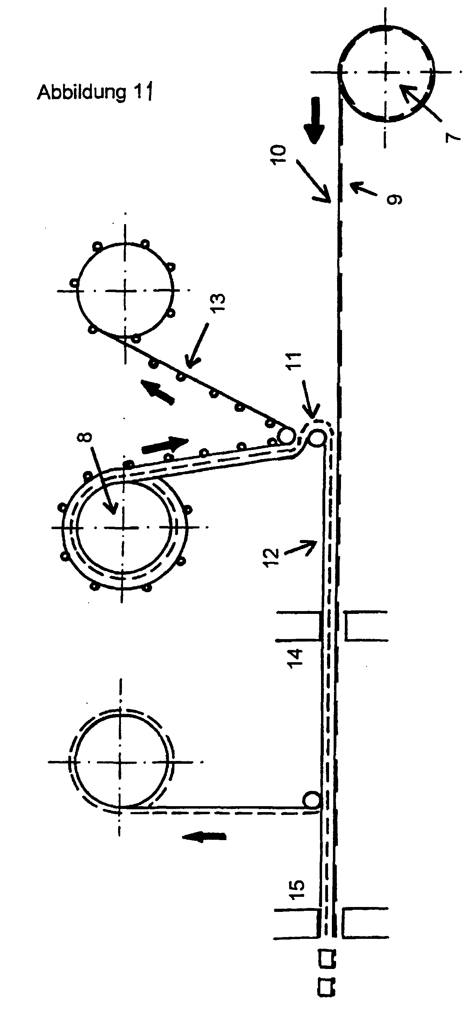

Die in diesem Beispiel beschriebene Verfahrensweise ist schematisch in Abbildung 11 wiedergegeben.

## Patentansprüche

1. Verbundlaminat aus mindestens zwei verschiedenen Schichten, wobei eine erste Schicht eine feuchtigkeitsempfindliche Schicht (1) ist, die ein feuchtigkeitsempfindliches polymeres Material, welches sich bei Einwirkung von Wasser löst, quillt oder chemisch umwandelt, und/oder einen feuchtigkeitsempfindlichen pharmazeutischen Wirkstoff, welcher bei Einwirkung von Wasser eine physikalische und/oder chemische Umwandlung erleiden kann, enthält, und eine zweite dieser Schichten eine feuchtigkeitsabsorbierende Schicht (2) ist, die ein Trockenmittel in einem aktiven Zustand enthält, **dadurch gekennzeichnet, dass** eine vertikale Trennschicht (4) oder eine horizontale Trennschicht (3), die für Feuchtigkeit undurchlässig oder für Feuchtigkeit durchlässig, jedoch für den feuchtigkeitsempfindlichen pharmazeutischen Wirkstoff undurchlässig ist, eine räumliche Trennung der feuchtigkeitsempfindlichen Schicht (1) von der feuchtigkeitsabsorbierenden Schicht (2) bewirkt.

2. Verbundlaminat nach Anspruch 1, **dadurch gekennzeichnet, dass** das feuchtigkeitsempfindliche polymere Material aus der Gruppe Polyvinylacetat, Polyvinylalkohol, Polyvinylpyrrolidon, Polyanhydrid, Cellulose, Celluloseprodukte und in Wasser quellbare Polymere ausgewählt ist.

3. Verbundlaminat nach einem der vorangegangenen Ansprüche, **dadurch gekennzeichnet, dass** der feuchtigkeitsempfindliche pharmazeutische Wirkstoff aus der Gruppe Acetylsalicylsäure, 17β-Estradiol, Nicotin, Morphin, Lobelin, Norethisteronacetat, Fentanyl, Selegilin, Testosteron und hydrolyseempfindliche Ester ausgewählt ist.

4. Verbundlaminat nach einem der vorangegangenen Ansprüche, **dadurch gekennzeichnet, dass** die feuchtigkeitsabsorbierende Schicht (2) mindestens einseitig haftklebend ist.

5. Verbundlaminat nach einem der vorangegangenen Ansprüche, **dadurch gekennzeichnet, dass** das Trockenmittel eine Einzelsubstanz oder eine Mischung aus der Gruppe Silicagel, Molekularsieb, Na₂SO₄, Na₂CO₃, CaSO₄, Al₂O₃, CaO, K₂CO₃ ist.

6. Verbundlaminat nach einem der vorangegangenen Ansprüche, **dadurch gekennzeichnet, dass** es ein Transdermales Therapeutisches System ist.

7. Verfahren zur Herstellung eines Verbundlaminats des Anspruchs 1 aus mindestens einer feuchtigkeitsempfindlichen Schicht (1), die ein feuchtigkeitsempfindliches polymeres Material und/oder einen feuchtigkeitsempfindlichen pharmazeutischen Wirkstoff enthält, und mindestens einer feuchtigkeitsabsorbierenden Schicht (2), die ein Trockenmittel in einem aktiven Zustand enthält, wobei in zwei Teilschritten eine feuchtigkeitsabsorbierende Schicht (2) und eine feuchtigkeitsempfindliche Schicht (1) auf eine Trägerschicht aufgetragen werden.

8. Verfahren zur Herstellung eines Verbundlaminats nach Anspruch 7, **dadurch gekennzeichnet, dass** die zwei Teilschritte zeitlich und/oder räumlich aufeinander folgend stattfinden.

9. Verfahren nach einem der Ansprüche 7 oder 8, **dadurch gekennzeichnet, dass** die feuchtigkeitsabsorbierende Schicht (2) und die feuchtigkeitsempfindliche Schicht (1) auf entgegengesetzten Seiten der Trägerschicht oder auf derselben Seite der Trägerschicht aufgetragen werden.

10. Verfahren nach einem der Ansprüche 7 bis 9, **dadurch gekennzeichnet, dass** in einem weiteren Schritt vor oder nach der Herstellung des Verbundlaminats aus feuchtigkeitsabsorbierender Schicht (2), Trägerschicht und feuchtigkeitsempfindlicher Schicht (1) die feuchtigkeitsabsorbierende Schicht (2) aktiviert wird.

11. Verfahren nach einem der Ansprüche 7 bis 10, **dadurch gekennzeichnet, dass** in einem weiteren Schritt nach Herstellung des Verbundlaminats aus feuchtigkeitsabsorbierender Schicht (2), Trägerschicht und feuchtigkeitsempfindlicher Schicht (1) durch Stanzen oder Schneiden einzelne Stanzlinge hergestellt werden.

12. Verfahren nach Anspruch 11, **dadurch gekennzeichnet, daß** die einzelnen Stanzlinge zusätzlich auf einen Tiefziehblister oder eine Siegelbahn übertragen und mit einer Siegelbahn abgedeckt, versiegelt und geschnitten werden.

## Claims

1. A composite laminate comprising at least two different layers, wherein a first layer is a moisture sensitive layer (1) comprising a moisture sensitive polymeric material, which dissolves, swells or chemically transforms under the influence of water and/or a moisture sensitive pharmaceutically active substance which physically and/or chemically transforms under the influence of water, and a second layer of these layers is a moisture absorbing layer (2) comprising a desiccant in an activated state, **characterized in that** a vertical separating layer (4) or a horizontal separating layer (3), which is impermeable for moisture or permeable for moisture but impermeable for the moisture sensitive pharmaceutically active substance, brings about a spatial separation of the moisture sensitive layer (1) from the moisture absorbing layer (2).

2. A composite laminate according to claim 1, wherein the moisture sensitive polymeric material is selected from the group consisting of polyvinyl acetate, polyvinyl alcohol, polyvinyl pyrrolidone, polyanhydride, cellulose, cellulose products and in water swellable polymers.

3. A composite laminate according to one of the preceding claims, wherein the moisture sensitive pharmaceutically active substance is selected from the group consisting of acetylsalicylic acid, 17β-estradiol, nicotine, morphine, lobeline, norethisterone acetate, fentanyle, selegiline, testosterone and hydrolytically sensitive esters.

4. A composite laminate according to one of the preceding claims, wherein the moisture absorbing layer (2) is adhesive on at least one side.

5. A composite laminate according to one of the preceding claims, wherein the desiccant is a single substance or a mixture from the group consisting of silica gel, molecular sieve, Na₂SO₄, Na₂CO₃, CaSO₄, Al₂O₃, CaO, K₂CO₃.

6. A composite laminate according to one of the preceding claims, which is a transdermal therapeutic system.

7. A process for producing a composite laminate of claim 1 comprising at least one moisture sensitive layer (1) which comprises a moisture sensitive polymeric material and/or a moisture sensitive pharmaceutically active substance, and at least one moisture absorbing layer (2) which comprises a desiccant in an activated state, wherein in two separate steps a moisture absorbing layer (2) and a moisture sensitive layer (1) are applied to a carrier layer.

8. A process for producing a composite laminate according to claim 7, wherein the two separate steps are carried out temporally and/or spatially consecutive.

9. A process according to claim 7 or 8, wherein the moisture absorbing layer (2) and the moisture sensitive layer (1) are applied to opposite sides of the carrier layer or to the same side of the carrier layer.

10. A process according to one of claims 7 to 9, wherein in a further step prior to or after manufacture of the composite laminate comprising the moisture absorbing layer (2), carrier layer and the moisture sensitive layer (1), the moisture absorbing layer (2) is activated.

11. A process according to one of claims 7 to 10, wherein in a further step following manufacture of the composite laminate comprising the moisture absorbing layer (2), carrier layer and the moisture sensitive layer (1), individual punched products are produced by punching or cutting.

12. A process according to claim 11, wherein the individual punched products are further transferred to a thermoformed blister or a sealable web and are covered with a sealable web, sealed and cut.

## Revendications

1. Stratifié composite composé d'au moins deux couches différentes, une première couche étant une couche sensible à l'humidité (1) contenant un matériau polymère sensible à l'humidité, lequel matériau se dissout, gonfle ou se modifie chimiquement sous l'action de l'eau, et/ou un principe actif pharmaceutique sensible à l'humidité capable de subir sous l'action de l'eau une modification physique et/ou chimique, et une deuxième de ces couches étant une couche absorbant l'humidité (2) qui contient un agent dessiccatif dans un état actif, **caractérisé en ce qu'**une couche de séparation verticale (4) ou une couche de séparation horizontale (3) qui est imperméable à l'humidité ou bien perméable à l'humidité mais imperméable au principe actif pharmaceutique sensible à l'humidité, assure une séparation dans l'espace de la couche sensible à l'humidité (1) d'avec la couche absorbant l'humidité (2).

2. Stratifié composite selon la revendication 1, **caractérisé en ce que** le matériau polymère sensible à l'humidité est choisi dans le groupe constitué par le polyacétate de vinyle, l'alcool polyvinylique, la polyvinylpyrrolidone, le polyanhydride, la cellulose, des produits cellulosiques et des polymères gonflant dans l'eau.

3. Stratifié composite selon l'une des revendications précédentes, **caractérisé en ce que** le principe actif pharmaceutique sensible à l'humidité est choisi dans le groupe constitué par l'acide acétylsalicylique, le 17β-oestradiol, la nicotine, la morphine, la lobéline, l'acétate de noréthistérone, le fentanyl, la sélégiline, la testostérone et des esters sensibles à l'hydrolyse.

4. Stratifié composite selon l'une des revendications précédentes, **caractérisé en ce que** la couche absorbant l'humidité (2) est adhésive sur au moins une de ses faces.

5. Stratifié composite selon l'une des revendications précédentes, **caractérisé en ce que** l'agent dessiccatif est une substance unique ou un mélange de substances choisies dans le groupe constitué par le gel de silice, un tamis moléculaire, Na₂SO₄, Na₂CO₃, CaSO₄, Al₂O₃, CaO, K₂CO₃.

6. Stratifié composite selon l'une des revendications précédentes, **caractérisé en ce qu'**il s'agit d'un système thérapeutique percutané.

7. Procédé de fabrication d'un stratifié composite selon la revendication 1 composé d'au moins une couche sensible à l'humidité (1) contenant un matériau polymère sensible à l'humidité et/ou un principe actif pharmaceutique sensible à l'humidité et d'au moins une couche absorbant l'humidité (2) contenant un agent dessiccatif dans un état actif, dans lequel une couche absorbant l'humidité (2) et une couche sensible à l'humidité (1) sont déposées en deux étapes partielles sur une couche support.

8. Procédé de fabrication d'un stratifié composite selon la revendication 7, **caractérisé en ce que** les deux étapes partielles s'effectuent à la suite l'une de l'autre dans le temps et/ou dans l'espace.

9. Procédé selon l'une des revendications 7 ou 8, **caractérisé en ce que** la couche absorbant l'humidité (2) et la couche sensible à l'humidité (1) sont déposées sur des faces opposées de la couche support ou bien sur la même face de la couche support.

10. Procédé selon l'une des revendications 7 à 9, **caractérisé en ce que** la couche absorbant l'humidité (2) est activée dans une autre étape antérieure ou postérieure à la fabrication du stratifié composite composé de la couche absorbant l'humidité (2), de la couche support et de la couche sensible à l'humidité (1).

11. Procédé selon l'une des revendications 7 à 10, **caractérisé en ce que** des découpes séparées sont produites par poinçonnage ou découpage dans une autre étape postérieure à la fabrication du stratifié composite composé de la couche absorbant l'humidité (2), de la couche support et de la couche sensible à l'humidité (1).

12. Procédé selon la revendication 11, **caractérisé en ce que** les différentes découpes sont en plus transférées sur un blister embouti ou sur une feuille thermoscellable et recouvertes d'une feuille thermoscellable, scellées et coupées.
